Europäisches Patentamt

European Patent Office

Office européen des brevets

(11) Publication number: **0 305 615**
**A1**

# EUROPEAN PATENT APPLICATION

(21) Application number: **87307775.4**

(22) Date of filing: **03.09.87**

(51) Int. Cl.⁴: **C07K 7/06 , C07K 7/08 ,**
**C07K 5/10 , C07K 5/08 ,**
**A61K 37/64**

(43) Date of publication of application:
**08.03.89 Bulletin 89/10**

(84) Designated Contracting States:
**AT BE CH DE FR GB IT LI NL SE**

(71) Applicant: **Immunetech Pharmaceuticals, Inc.**
**11045 Roselle Street**
**San Diego, California 92 121(US)**

(72) Inventor: **Hahn, Gary Scott**
**2371 Lagoon View Drive**
**Cardiff by the Sea California 92007(US)**

(74) Representative: **Armitage, Ian Michael et al**
**MEWBURN ELLIS & CO. 2/3 Cursitor Street**
**London EC4A 1BQ(GB)**

(54) Peptide antagonists for the C5a anaphylatoxin.

(57) Peptides and peptide compositions are disclosed which act as competitive antagonists for the C5a receptor and thus block inflammatory and immunomodulatory activity mediated by endogenous C5a.

EP 0 305 615 A1

## PEPTIDE ANTAGONISTS FOR THE C5a ANAPHYLATOXIN

The invention relates to peptides which bind to and block the C5a anaphylatoxin activity.

The general inflammatory response of humans occurs whenever tissue is injured. The injury may be due to a wide variety of conditions including infection by bacteria, viruses or fungi, invasion by cancer cells, allergic or autoimmune diseases and physically- or chemically-induced trauma. In all of these diseases and conditions, the complement system is activated resulting in production of C5a which serves to amplify and exacerbate the resulting inflammation. By binding to and blocking the C5a receptor, the peptides of the present invention can reduce or prevent C5a-mediated inflammation. In addition, these peptides may inhibit the immunoregulatory effects of C5a and thus indirectly cause immunoregulation.

The anaphylatoxin C5a is one of the more potent biologic factors known to man. This factor is generated when C5 is cleaved at a specific site by convertases (proteolytic enzymes) of the blood complement system, as well as by enzymes of the coagulation system. C5a is the last in a series of three anaphylatoxins that are released during activation of the complement cascade. Anaphylatoxins C3a and C4a, factors released prior to C5a in the activation sequence, are considerably less potent than C5a for inducing cellular histamine release and tissue spasmogenic responses, and for enhancing vascular permeability. Furthermore, C5a is the most active leukotactic respiratory distress syndrome, idiopathic pulmonary fibrosis ad immune complex-mediated glomerulonephritis.

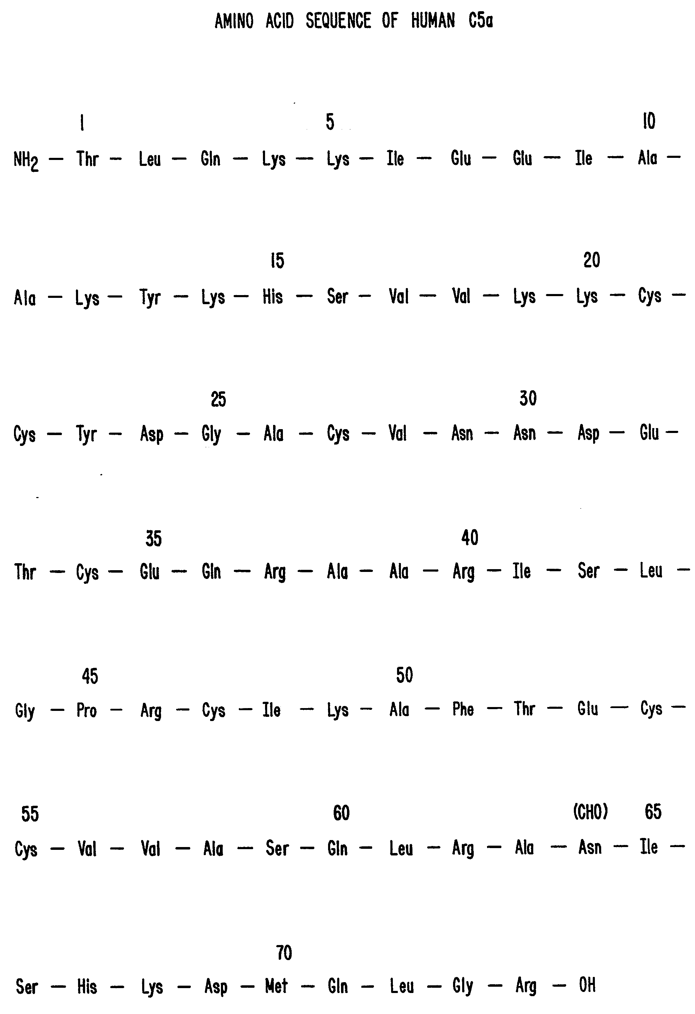

Figure 1 illustrates the amino acid sequence of native human C5a.

Attempts to identify the submolecular site(s) in C5a which directly bind to the C5a receptor have produced only incomplete results. The complex oligosaccharide moiety which is linked to asparagine 64 of native C5a does not appear to form part of the C5a receptor binding site. Removal of the oligosaccharide moiety from native C5a does not change its biological activity. Surprisingly, deglycosylated C5a des-Arg-74 is approximately tenfold more active than native C5a des-Arg-74, indicating that the oligosaccharide may partially block or otherwise negatively modulate the bioactivity of C5a des-Arg. Enzymatic removal by carboxypeptidase B of the C-terminal arginine residue from the 74 residue C5a chain (Figure 1) to produce C5a des-Arg-74 resulted in reduction but not abolition of the bioactivity of C5a, indicating that the C-terminus of the molecule was important but not required for activity. This finding is in contrast to the bioactivity of the C3a and C4a anaphylatoxins which are inactivated after removal of their C-terminal arginine. The bioactivity of C5a des-Arg-74 is further reflected by its ability to bind to the neutrophil C5a receptor and to compete effectively with native C5 for its receptor. Enzymatic removal of the five C-terminal residues from human C5a produces a biofunctionally inactive derivative, C5a 1-69, which continues to exhibit substantial C5a receptor binding activity. The ability of C5a 1-69 to act as a competitive antagonist with C5a for its receptor indicates that a second receptor binding factor known to originate from any blood component.

Upon release, C5a binds to its specific cellular receptors present on blood leukocytes including polymorphonuclear neutrophils, monocytes, basophils, and eosinophils, and to tissue-resident cells such as macrophages and mast cells. Such binding of C5a to its receptor triggers cells to release vasoactive and inflammatory substances including histamine, leukotrienes, prostaglandins, and proteolytic enzymes. These and other substances produce contraction of smooth muscle, platelet aggregation, vasodilation, and increased vascular permeability which results in the pain and swelling which is characteristic of inflammation. In addition, C5a is a powerful attractant of leukocytes to sites of inflammation by the process of chemotaxis which leads to an increased cellular inflammatory response. C5a also exerts immunodulatory effects on immune responsiveness as evidenced by its ability to potentiate the primary antibody response to antigens in vitro.

The present invention provides peptides and peptide compositions which act as competitive antagonists for the C5a receptor and thus block inflammatory and immunomodulatory activity mediated by endogenous C5a.

Although the peptides of the present invention can act as inhibitors of inflammation due to many causes, these peptides are particularly useful in the treatment of diseases or conditions in which acute or chronic inflammation significantly contributes to disease symptoms and disease pathogenesis. Such diseases or conditions include, but are not limited to, autoimmune diseases including rheumatoid arthritis and systemic lupus erythematosus, vasculitis, serum sickness, angioedema, bullous skin diseases, hypersensitivity pneumonitis, adult site of C5a distinct from the carboxyterminus exists.

Synthetic peptides with sequences identical to portions of the C5a C-terminus exhibit no bioactivity when compared to intact C5a and cannot effectively act as competitive antagonists for the C5a receptor when mixed with C5a in receptor binding or anaphylatoxin bioassays.

These results suggest that the carboxy-terminal region of C5a contains residues primarily responsible for activating or triggering the C5a receptor while a separate, distinct receptor-binding site having minimal receptor activating capacity exists in portions of the molecule closer to the amino-terminus.

Studies of cyanogen bromide-cleaved porcine C5a indicate that a peptide identical to C5a amino acid sequences (aa) 2-17 has negligible C5a biological activity while a fragment representing aa 18-73 had < 1% of the activity of intact C5a, indicating that the first 17 residues are not critical for C5a function.

Nitration of Tyrosine-23 of C5a des-Arg-74 substantially reduced the peptide's neutrophil binding activity, providing further evidence for the importance of amino-terminal residues for receptor binding. From this data alone, however, it is unclear whether the tyrosine in question directly binds to receptor structures or whether the chemical modification results in distortion of the C5a three-dimensional structure or perturbation of other critical residues spatially related to the tyrosine.

In the following description, the amino acid components of the peptides are identified as abbreviations for convenience. These abbreviations are intended to include both the D- and L-forms, although the L-form is preferred:

| Amino Acid | Abbreviation |
|---|---|
| glycine | Gly |
| alanine | Ala |
| valine | Val |
| leucine | Leu |
| isoleucine | Ile |
| proline | Pro |
| methionine | Met |
| cysteine | Cys |
| phenylalanine | Phe |
| tyrosine | Tyr |
| tryptophan | Trp |
| histidine | His |
| lysine | Lys |
| arginine | Arg |
| aspartic acid | Asp |
| asparagine | Asn |
| glutamic acid | Glu |
| glutamine | Gln |
| serine | Ser |
| threonine | Thr |
| ornithine | Orn |

Surprisingly, the present invention provides peptides and peptide compositions which act as competitive antagonists for the C5a receptor and thus block inflammatory and immunomodulatory activity mediated by endogenous C5a. In its broadest scope, the present invention provides peptides having the following formula:

A-B-C-R-D-E-F

where

A is H (hydrogen) or an amino acid or polypeptide selected from the following list:

Lys-His-Ser-Val-Val-Lys-Lys-

His-Ser-Val-Val-Lys-Lys-

Ser-Val-Val-Lys-Lys-

Val-Val-Lys-Lys-

Val-Lys-Lys-

Lys-Lys-

Lys-

B is H (hydrogen) or an amino acid selected from Cys and Ser; B is H (hydrogen) or an amino acid selected from Cys and Ser; R is a "core" tetrapeptide:
Tyr-Asp-Gly-Ala or Asp-Gly-Ala-Tyr
or a "core" tripeptide:
Asp-Gly-Ala
D is OH (hydroxyl) or an amino acid selected from Cys, Ser and Tyr; E is OH (hydroxyl) or an amino acid selected from Val and Arg; and F is OH (hydroxyl) or an amino acid or polypeptide selected from the following list:

Asn-Asn-Asp-Glu-Thr-Cys-Glu-Gln-Arg

Asn-Asn-Asp-Glu-Thr-Cys-Glu-Gln

Asn-Asn-Asp-Glu-Thr-Cys-Glu

Asn-Asn-Asp-Glu-Thr-Cys

Asn-Asn-Asp-Glu-Thr

Asn-Asn-Asp-Glu

Asn-Asn-Asp

Asn-Asn

Asn

or peptides identical to the first four listed in which Ser is substituted for Cys. As will be discussed below, derivatives of the foregoing peptides may also be used in the practice of the present invention.

It is evident from this description that the invention consists of an essential "core" tetrapeptide (Tyr-Asp-Gly-Ala or Asp-Gly-Ala-Tyr, or a "core" tripeptide Asp-Gly-Ala, which displays C5a blocking activity. This blocking activity is maintained or increased by amino- and/or carboxy-terminal additions of amino acid or polypeptide moieties which are identical to those found in the native human C5a sequence from aa 14-37.

In addition, it has been discovered that certain amino acid sequence positions in the peptides of the present invention could be substituted with amino acids not found in the native human C5a molecule. For example, all Cys residues can be substituted with Ser, an amino acid which closely resembles Cys in size and side chain configuration. The Cys corresponding to Cys-27 of native human C5a can, in addition, be substituted with Tyr, a residue which occurs at the corresponding position of porcine C5a. Similarly, the Val corresponding to Val-28 of the native human C5a sequence can also be substituted with Arg, a residue which occurs at the corresponding position of porcine C5a. For example, the following peptides which display C5a receptor blocking activity illustrate peptides of the present invention.

4

Asp-Gly-Ala-Cys-Val-Asn-Asn-Asp-Glu

Tyr-Asp-Gly-Ala-Cys-Val-Asn-Asn-Asp-Glu

Ser-Ser-Tyr-Asp-Gly-Ala-Cys-Val-Asn-Asn-Asp-Glu

Lys-Ser-Ser-Tyr-Asp-Gly-Ala-Cys-Val-Asn-Asn-Asp-Glu

Lys-Lys-Ser-Ser-Tyr-Asp-Gly-Ala-Cys-Val-Asn-Asn-Asp-Glu

Val-Lys-Lys-Ser-Ser-Tyr-Asp-Gly-Ala-Cys-Val-Asn-Asn-Asp-Glu

Val-Val-Lys-Lys-Ser-Ser-Tyr-Asp-Gly-Ala-Cys-Val-Asn-Asn-Asp-Glu

Ser-Val-Val-Lys-Lys-Ser-Ser-Tyr-Asp-Gly-Ala-Cys-Val-Asn-Asn-Asp-Glu

His-Ser-Val-Val-Lys-Lys-Ser-Ser-Tyr-Asp-Gly-Ala-Cys-Val-Asn-Asn-Asp-Glu

Lys-His-Ser-Val-Val-Lys-Lys-Ser-Ser-Tyr-Asp-Gly-Ala-Cys-Val-Asn-Asn-Asp-Glu

Asp-Gly-Ala-Cys-Val-Asn-Asn-Asp-Glu

Tyr-Asp-Gly-Ala-Cys-Val-Asn-Asn-Asp-Glu

Lys-Lys-Ser-Ser-Tyr-Asp-Gly-Ala-Cys-Val-Asn-Asn-Asp-Glu

Lys-His-Ser-Val-Val-Lys-Lys-Ser-Ser-Tyr-Asp-Gly-Ala-Cys-Val-Asn-Asn-Asp-Glu

Asp-Gly-Ala

Tyr-Asp-Gly-Ala

Cys-Tyr-Asp-Gly-Ala

Cys-Cys-Tyr-Asp-Gly-Ala

Lys-Cys-Cys-Tyr-Asp-Gly-Ala

Asp-Gly-Ala-Tyr-Arg

Tyr-Asp-Gly-Ala-Tyr-Arg

Ser-Tyr-Asp-Gly-Ala-Tyr-Arg

Ser-Ser-Tyr-Asp-Gly-Ala-Tyr-Arg

Lys-Ser-Ser-Tyr-Asp-Gly-Ala-Tyr-Arg

Lys-Lys-Ser-Ser-Tyr-Asp-Gly-Ala-Tyr-Arg

Asp-Gly-Ala-Ser

Ser-Tyr-Asp-Gly-Ala-Ser

Ser-Ser-Tyr-Asp-Gly-Ala-Ser

Lys-Ser-Ser-Tyr-Asp-Gly-Ala-Ser

Lys-Lys-Ser-Ser-Tyr-Asp-Gly-Ala-Ser

Lys-Cys-Cys-Tyr-Asp-Gly-Ala-Cys-Val-Asn-Asn-Asp-Glu-Thr-Cys-Glu-Gln-Arg

Cys-Cys-Tyr-Asp-Gly-Ala-Cys-Val-Asn-Asn-Asp-Glu-Thr-Cys-Glu-Gln-Arg

Cys-Tyr-Asp-Gly-Ala-Cys-Val-Asn-Asn-Asp-Glu-Thr-Cys-Glu-Gln-Arg

Tyr-Asp-Gly-Ala-Cys-Val-Asn-Asn-Asp-Glu-Thr-Cys-Glu-Gln-Arg

Asp-Gly-Ala-Cys-Val-Asn-Asn-Asp-Glu-Thr-Cys-Glu-Gln-Arg

Gly-Ala-Cys-Val-Asn-Asn-Asp-Glu-Thr-Cys-Glu-Gln-Arg

Ala-Cys-Val-Asn-Asn-Asp-Glu-Thr-Cys-Glu-Gln-Arg

Asn-Asn-Asp-Glu-Thr-Cys-Glu-Gln-Arg

Asp-Glu-Thr-Cys-Glu-Gln-Arg

Thr-Cys-Glu-Gln-Arg

Cys-Glu-Gln-Arg

Certain other amino acids may be substituted for those listed so long as they are "functionally conserved" in the sense that they preserve or increase the C5a receptor-blocking activities which characterize the peptides of the present invention. That is, amino acids which are chemically similar by virtue of similar side chain size, charge, shape, solubility and related characteristics may be substituted for those listed while retaining the peptide's biological activity. Groups of amino acids characterized by such chemical similarity and retention of biological activity are termed "functionally conserved," as proposed by Margaret Dayhoff, et al., "A Model of Evolutionary Change in Proteins" in the Atlas of Protein Sequence and Structure, Volume 5 (1972), published by the National Biomedical Research Foundation, and incorporated herein by reference. Functionally conserved groups of amino acids include:

| Group | Amino Acids |
|---|---|
| small aliphatic | Ala, Pro, Gly |
| acid amide | Gln, Asn |
| acid | Glu, Asp |
| hydroxyl | Ser, Thr |
| sulfhydryl | Cys |
| aliphatic | Val, Ile, Leu, Met |
| basic | Lys, Arg, His |
| aromatic | Phe, Tyr, Trp |

Functionally conserved groups of amino acids need not be limited to the 20 amino acids listed above. Other amino acids having chemical properties and biological effects similar to one of the eight groups listed above may also be substituted within a particular group for the listed amino acids. For example, ornithine and homoarginine have basic side chains similar to Lys, Arg, and His and may be substituted therefor while retaining the peptide's biological activity. In addition, it is within the scope of the present invention that other moieties such as fatty acids or lipophilic polypeptides or amphipathic polypeptides which increase the ability of the peptides of the present invention to bind non-specifically to lipids of cell surface membranes may be covalently added to either the amino- or carboxy-terminal ends of the C5a-blocking peptides to increase their activity.

Within the general sequence
A-B-C-R-D-E-F
it is preferred that:

A is Val-Val-Lys-Lys,

Val-Lys-Lys,

Lys-Lys, or

Lys

B is Ser

C is Ser

D is Ser or Tyr

E is Val, and

F is Asn-Asn or Asn.

Various derivatives of the foregoing peptides may also be formed while preserving or augmenting the activity described for the peptides. For example, chemical substitution of acyl, alkyl and other substituents at the N-terminus, or formation of esters or amides at the C-terminus, may yield peptide derivatives with preserved or augmented activity. Preferred substituents at the N-terminus include aliphatic and aromatic acyl substituents of the form RCO-, as well as substituents of the form R-, where R is preferably an unbranched or branched alkyl group of from one to about eight carbons, and may also be $C_2$-$C_8$ alkenyl, $C_2$-$C_8$ alkynyl, $C_6$-$C_{14}$ aryl, $C_7$-$C_{14}$ alkaryl, $C_7$-$C_{14}$ aralkyl or $C_3$-$C_{14}$ cycloalkyl. Similarly, the C-terminus of the peptides may be amidated or esterified with a substituent of the form -NHR, -NR$_2$ or -OR, wherein R is defined as above.

A number of assays may have performed using representative peptides of the prevent invention, and indicate that these peptides have utility as described herein. One such assay measures the chemiluminescence associated with oxygen-containing radicals or other reactive intermediates released from neutrophils and other cells in response to the C5a anaphylatoxin. The assay is discussed in Trush, M.A., et al., "The Generation of Chemiluminescence (CL) by Phagocytic Cells," Methods of Enzymology, Vol. 57, pp. 463-495 (Academic Press 1978). The chemiluminescence assay measures the ability of the peptides of the present invention to inhibit the release of radicals and other reactive intermediates such as the hydroxyl radical, superoxide anion, singlet oxygen and hydrogen peroxide as released from leukocytes and other cells in response to C5a binding to specific cellular receptors on the cells. As demonstrated by the following representative chemiluminescence data, the peptides of the present invention do indeed possess such inhibitory activity.

### I. PEPTIDE: Asp-Gly-Ala-Tyr

Control: 2,300,000 counts
Peptide-Inhibited Sample: 550,000 counts
Percent Inhibition: 76%

### II. PEPTIDE: Tyr-Asp-Gly-Ala-Tyr-Arg

Control: 1,200,000 counts
Peptide-Inhibited Sample: 430,000 counts
Percent Inhibition: 64%

### III. PEPTIDE: Asp-Gly-Ala

Control: 726,000 counts
Peptide-Inhibited Sample: 180,000 counts
Percent Inhibition: 75%

The above assays were run in the presence of neutrophils, 0.3 ng/ml C5a (controls and peptide-inhibited samples) and 1.0 mM peptide (peptide-inhibited samples).

Other assays were performed using standard radioimmunoassay techniques to measure the ability of the subject peptides to act as antagonists to C5a. Using these assays, release of the enzyme myeloperoxidase from leukocytes and macrophages in response to C5a-mediated degranulation was measured. Also measured was release of the protein lactoferrin from polymorphonuclear neutrophils during C5a-mediated degranulation. The subject peptides were shown to inhibit release of these proteins, which directly reflects inhibition of C5a-mediated processes associated with inflammation, degranulative tissue destruction and certain autoimmune diseases. The following data are representative of such inhibition in response to subject peptides present in approximately 1 mM concentrations.

## I. PEPTIDE: Lys-Lys-Ser-Ser-Tyr-Asp-Gly-Ala-Tyr-Arg

Myeloperoxidase Inhibition: 22.9%
Lactoferrin Inhibition: 8.7%

## II. PEPTIDE: Tyr-Asp-Gly-Ala-Tyr-Arg

Myeloperoxidase Inhibition: 22.4%
Lactoferrin Inhibition: 28.4%

## III. PEPTIDE: Tyr-Asp-Gly-Ala-Tyr

Myeloperoxidase Inhibition: 48.4%
Lactoferrin Inhibition: 72.5%

## IV. PEPTIDE: Asp-Gly-Ala-Tyr

Myeloperoxidase Inhibition: 100%
Lactoferrin Inhibition: 100%

## V. PEPTIDE: Asp-Gly-Ala

Myeloperoxidase Inhibition: 22.6%
Lactoferrin Inhibition: 20.2%

Useful pharmaceutical carriers for the preparation of the compositions hereof can be solids, liquids or gases; thus, the compositions can take the form of tablets, pills, capsules, powders, enterically coated or other protected formulations (such as by binding on ion exchange resins or other carriers, or packaging in lipid-protein vesicles or adding additional terminal amino acids or replacing a terminal amino acid in the L-form with one in the D-form), sustained release formulations, solutions (e.g., ophthalmic drops), suspensions, elixirs, aerosols, and the like. The carrier can be selected from various oils including those of petroleum, animal, vegetable or synthetic origin, as for example, peanut oil, soybean oil, mineral oil, sesame oil and the like. Water, saline, aqueous dextrose and glycols are preferred liquid carriers, particularly (when isotonic) for injectable solutions. Suitable pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like. The compositions may be subjected to conventional pharmaceutical expedients such as sterilization and may contain conventional pharmaceutical additives such as preservatives, stabilizing agents, wetting or emulsifying agents, salts for adjusting osmotic pressure, buffers, and the like. Suitable pharmaceutical carriers

8

and their formulation are described in "Remington's Pharmaceutical Sciences" by E. W. Martin. Such compositions will, in any event, contain an effective amount of the active compound together with a suitable amount of carrier so as to prepare the proper dosage form for proper administration to the host.

To be effective for the prevention or treatment of the inflammation responses it is important that the therapeutic agents be relatively non-toxic, non-antigenic and non-irritating at the levels in actual use.

Peptides of this invention may be synthesized by the solid phase peptide synthesis (or Merrifield) method. This established and widely used method is described, including experimental procedures, in the following references:

Merrifield, J. Am. Chem. Soc., 85, 2149-2154 (1963).

Meienhofer in "Hormonal Proteins and Peptides," ed. C.H. Li, Vol. 2 (Academic Press, 1973), pp. 48-267.

Barany and Merrifield in "The Peptides," eds. E. Gross and F. Meienhofer, Vol. 2 (Academic Press, 1980), pp. 3-285.

A preferred method for synthesizing the peptides of the present invention is the so-called "Merrifield" synthesis technique which is well known to those skilled in the art and is set forth in detail in the article entitled "Synthesis of a Tetrapeptide" by R.B. Merrifield, Journal of the American Chemical Society (Vol. 85, pp. 2149-2154 (1963)) as well as Meienhofer, cited above.

In this preferred method a peptide of any desired length and of any desired sequence is produced through the stepwise addition of amino acids to a growing peptide chain which is bound by a covalent bond to a solid resin particle.

In the preferred application of this method the C-terminal end of the growing peptide chain is covalently bound to a resin particle and amino acids having protected amino groups are added in the stepwise manner indicated above. A preferred amino protecting group is the t-BOC group, which is stable to the condensation conditions and yet is readily removable without destruction of the peptide bonds or racemization of chiral centers in the peptide chain. At the end of the procedure, the final peptide is cleaved from the resin, and any remaining protecting groups are removed, by treatment under acidic conditions such as, for example, with a mixture of hydrobromic acid and trifluoroacetic acid or with hydrofluoric acid, or the cleavage from the resin may be effected under basic conditions, for example, with triethylamine, the protecting groups then being removed under acid conditions.

The cleaved peptides are isolated and purified by means well known in the art such as, for example, lyophilization followed by either exclusion or partition chromatography on polysaccharide gel media such as Sephadex G25, or countercurrent distribution. The composition of the final peptide may be confirmed by amino acid analysis after degradation of the peptide by standard means.

Salts of carboxyl groups of the peptide may be prepared in the usual manner by contacting the peptide, with one or more equivalents of a desired base such as, for example, a metallic hydroxide base, e.g., sodium hydroxide; a metal carbonate or bicarbonate base such as, for example, sodium carbonate or sodium bicarbonate; or an amine base such as, for example, triethylamine, triethanolamine and the like. Acid addition salts of the polypeptides may be prepared by contacting the polypeptide with one or more equivalents of the desired inorganic or organic acid, such as, for example, hydrochloric acid. Such salts may be expected to retain the biological activity of the parent molecule.

Esters of carboxyl groups of the peptides may be prepared by any of the usual means known in the art for converting a carboxylic acid or precursor to an ester. One preferred method for preparing esters of the present peptides, when using the Merrifield synthesis technique describe above, is to cleave the completed peptide from the resin in the presence of the desired alcohol either under basic or acidic conditions, depending upon the resin. Thus, the C-terminal end of the peptide when freed from the resin is directly esterified without isolation of the free acid.

Amides of the polypeptides of the present invention may also be prepared by techniques well known in the art for converting a carboxylic acid group or precursor to an amide. A preferred method for amide formation at the C-terminal carboxyl group is to cleave the polypeptide from a solid support with an appropriate amine, or to cleave in the presence of an alcohol, yielding an ester, followed by aminolysis with the desired amine.

N-acyl derivatives of an amino group of the present polypeptides may be prepared by utilizing an N-acyl protected amino acid for the final condensation, or by acylating a protected or unprotected peptide. O-acyl derivatives·may be prepared, for example, by acylation of a free hydroxy peptide or peptide resin. Either acylation may be carried out using standard acylating reagents such as acyl halides, anhydrides, acyl imidazoles, and the like. Both N- and O-acylation may be carried out together, if desired.

The coupling, deprotection/cleavage reactions and preparations of derivatives of the subject polypeptides are suitably carried out at temperatures between about -10° and +50° C., most preferably about 20° - 25° C. The exact temperature for any particular reaction will of course be dependent upon the substrates, reagents, solvents and so forth, all being well within the skill of the practitioner.

It is understood that various other modifications will be apparent to and can readily be made by those skilled in the art without departing from the scope and spirit of this invention. Accordingly, it is not intended that the scope of the claims appended hereto be limited to the description as set forth herein, but rather that the claims be construed as encompassing all the features of patentable novelty which reside in the present invention, including all features which would be treated as equivalents thereof by those skilled in the art to which this invention pertains.

## Claims

1. A peptide antagonist for C5a activity, said peptide having an amino acid sequence A-B-C-R-D-E-F wherein:

A is an amino-terminal hydrogen or an amino acid or polypeptide selected from the following list:

Lys-His-Ser-Val-Val-Lys-Lys-

His-Ser-Val-Val-Lys-Lys-

Ser-Val-Val-Lys-Lys-

Val-Val-Lys-Lys-

Val-Lys-Lys-

Lys-Lys-

Lys-;

B is said amino-terminal hydrogen or an amino acid selected from Cys and Ser; C is said amino-terminal hydrogen or an amino acid selected from Cys and Ser; R is a "core" tetrapeptide:
Tyr-Asp-Gly-Ala or Asp-Gly-Ala-Tyr
or a "core" tripeptide:
Asp-Gly-Ala;
D is a carboxy-terminal hydroxyl or an amino acid selected from Cys, Ser and Tyr; E is said carboxy-terminal hydroxyl or an amino acid selected from Val and Arg; and F is said carboxy-terminal hydroxyl or an amino acid or polypeptide selected from the following list:

Asn-Asn-Asp-Glu-Thr-Cys-Glu-Gln-Arg

Asn-Asn-Asp-Glu-Thr-Cys-Glu-Gln

Asn-Asn-Asp-Glu-Thr-Cys-Glu

Asn-Asn-Asp-Glu-Thr-Cys

Asn-Asn-Asp-Glu-Thr

Asn-Asn-Asp-Glu

Asn-Asn-Asp

Asn-Asn

Asn

and polypeptides identical to the first four listed in which Ser is substituted for Cys; and pharmaceutically acceptable esters, amides, N-acyl and O-acyl derivatives and acid- and base-addition salts of the foregoing peptides.

2. The peptide antagonist of claim 1, wherein R is Tyr-Asp-Gly-Ala.

3. The peptide antagonist of claim 1, wherein R is Asp-Gly-Ala-Tyr.

4. The peptide antagonist of claim 1, wherein R is Asp-Gly-Ala.

5. The peptide antagonist of claim 1, 2, 3 or 4 wherein A is selected from the group consisting of

**Val-Val-Lys-Lys,**

**Val-Lys-Lys,**

**Lys-Lys, and**

**Lys.**

6. The peptide antagonist of claim 5, wherein A is selected from the group consisting of Lys-Lys and Lys.

7. The peptide antagonist of claim 1, 2, 3 or 4, wherein B is Ser.

8. The peptide antagonist of claim 1, 2, 3 or 4, wherein C is Ser.

9. The peptide antagonist of claim 1, 2, 3 or 4, wherein A is Lys-Lys or Lys, B is Ser, and C is Ser.

10. The peptide antagonist of claim 1, 2, 3 or 4, wherein D is selected from the group consisting of Ser and Tyr.

11. The peptide antagonist of claim 9, wherein D is selected from the group consisting of Ser and Tyr.

12. The peptide antagonist of claim 1, 2, 3 or 4, wherein E is Val.

13. The peptide antagonist of claim 1, 2, 3 or 4, wherein F is selected from the group consisting of Asn-Asn and Asn.

14. The peptide antagonist of claim 5, wherein F is selected from the group consisting of Asn-Asn and Asn.

15. The peptide antagonist of claim 9, wherein F is selected from the group consisting of Asn-Asn and Asn.

16. The peptide antagonist of claim 12, wherein F is selected from the group consisting of Asn-Asn and Asn.

17. Use of a peptide antagonist of claim 1 for the manufacture of a medicament for suppressing inflammation responses.

18. A process for the preparation of a peptide antagonist of claim 1 characterized in that such peptide is liberated from its corresponding functional derivative, which may be bound by a covalent bond to a solid resin, by treatment under acidic or basic conditions, and, if desired, the peptide thus obtained is esterified, amidated and/or acrylated to yield a corresponding ester, amide, N- and/or O-acyl derivative or is transformed into one of its pharmacologically acceptable salts by treatment with a base or an acid.

19. A pharmaceutical preparation comprising a peptide antagonist of claim 1 and at least one pharmaceutically acceptable carrier.

20. Use of a peptide antagonist of claim 1 for suppressing inflammation responses.

21. A process comprising preparation of a peptide antagonist according to any one of claims 1-17.

## AMINO ACID SEQUENCE OF HUMAN C5a

1                              5                              10
$NH_2$ — Thr — Leu — Gln — Lys — Lys — Ile — Glu — Glu — Ile — Ala —

                    15                             20
Ala — Lys — Tyr — Lys — His — Ser — Val — Val — Lys — Lys — Cys —

                    25                             30
Cys — Tyr — Asp — Gly — Ala — Cys — Val — Asn — Asn — Asp — Glu —

                    35                             40
Thr — Cys — Glu — Gln — Arg — Ala — Ala — Arg — Ile — Ser — Leu —

               45                             50
Gly — Pro — Arg — Cys — Ile — Lys — Ala — Phe — Thr — Glu — Cys —

55                                   60                    (CHO)   65
Cys — Val — Val — Ala — Ser — Gln — Leu — Arg — Ala — Asn — Ile —

               70
Ser — His — Lys — Asp — Met — Gln — Leu — Gly — Arg — OH

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 67, 1967, page 6111, abstract no. 64706a, Columbus, Ohio, US; & AT-B-250 572 (GEDEON RICHTER VEGYESZETI GYAR R.T.) 25-11-1966 * Abstract * | 1 | C 07 K   7/06 C 07 K   7/08 C 07 K   5/10 C 07 K   5/08 A 61 K  37/64 |
| A | CHEMICAL ABSTRACTS, vol. 101, 1984, page 510, abstract no. 168770d, Columbus, Ohio, US; F. MARCEAU et al.: "Effect of C3a and C5a anaphylatoxins on guinea pig isolated blood vessels", & J. PHARMACOL. EXP. THER. 1984, 230(3), 749-54 * Abstract * | 1,18,19 | |
| A | CHEMICAL ABSTRACTS, vol. 106, 1987, page 243, abstract no. 45817v, Columbus, Ohio, US; T. GEIGER et al.: "Deamidation, isomerization, and racemization at asparaginyl and aspartyl residues in peptides. Succinimide-linked reactions that contribute to protein degradation", & J. BIOL. CHEM. 1987, 262(2), 785-94 * Abstract * -/- | 1 | **TECHNICAL FIELDS SEARCHED (Int. Cl.4)** C 07 K   7/00 C 07 K   5/00 A 61 K  37/00 |

The present search report has been drawn up for all claims

| Place of search | Date of completion of the search | Examiner |
|---|---|---|
| THE HAGUE | 25-04-1988 | RAJIC M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

**EUROPEAN SEARCH REPORT**

Application Number

EP 87 30 7775

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 102, 1985, page 303, abstract no. 108710v, Columbus, Ohio, US; C.M. O'CONNOR et al.: "Mammalian brain and erythrocyte carboxyl methyltransferases are similar enzymes that recognize both D-aspartyl and L-isoaspartyl residues in structurally altered protein substrates", & PROC. NATL. ACAD. SCI. U.S.A. 1984, 81(24), 7757-61 * Abstract * | 1 | |
| .L | US-A-4 692 511 (HAHN) * Whole document * | 1-21 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 25-04-1988 | RAJIC M. |

EPO FORM 1503 03.82 (P0401)